# EUROPEAN PATENT SPECIFICATION

(11) **EP 4 093 336 B1**
(45) Date of publication and mention of the grant of the patent: **07.01.2026**
(21) Application number: 21705776.9
(22) Date of filing: 21.01.2021
(51) Int. Cl.: A61F 2/40, A61F 2/30

(54) **MOBILE BEARING REVERSED HUMERAL IMPLANT**
BEWEGLICH GELAGERTES INVERSES HUMERUSIMPLANTAT
IMPLANT HUMÉRAL REVERSÉ À PLATEAU MOBILE

(30) Priority: 24.01.2020 US 202062965581 P
(43) Date of publication of application: 30.11.2022
(73) Proprietor: Zimmer, Inc., Warsaw, Indiana 46580 (US)
(72) Inventor: HOPKINS, Andrew Rolfe, 8404 Winterthur (CH)
(74) Representative: Allen, Caroline Margaret
(86) International application number: PCT/US2021/014380
(87) International publication number: WO 2021/150730

(56) References cited:
- FR-A1- 2 825 263
- FR-A1- 2 992 165
- GB-A- 2 166 654
- US-A1- 2012 004 733

## Description

### FIELD

The present subject matter relates to an orthopedic system and specifically to a shoulder implant system.

### BACKGROUND

In a healthy shoulder, the proximal humerus is generally ball-shaped, and articulates within a socket formed by the scapula, called the glenoid, to form the shoulder joint. Some implant systems for the total replacement of the shoulder joint generally replicate the natural anatomy of the shoulder. Such implant systems can include a humeral component having a stem that fits within the humeral canal, and an articulating head that articulates within the socket of a glenoid component implanted within the glenoid of the scapula.

Reverse-type shoulder implant systems have been developed in which the conventional ball-and-socket configuration that replicates the natural anatomy of the shoulder is reversed, such that a concave recessed articulating component is provided at the proximal end of the humeral component that articulates against a convex portion of a glenosphere of a glenoid component.

After a reverse-type shoulder operation some patients find that the functional outcome is impaired, and some daily tasks are difficult to perform.

GB2166654 discloses a reverse shoulder prosthesis consisting of a bowl-shaped humeral tray, a glenosphere and a mobile insert located between the tray and the glenosphere.

FR2992165A1 discloses an implant having a base part including a cavity opening into a proximal face. A joint part includes a joint surface, a planar sliding surface, and a stud. The height and the cross-section of the stud are lower than the depth and the cross-section of the cavity such that the stud is committed in the cavity until the arrival of the sliding surface in contact with the proximal face, to allow mobility of the joint part relative to the base part within the limit of the arrival of the stud in contact with walls of the base part delimiting the cavity.

The present invention provides an apparatus, as defined in claim 1. Further optional features of the invention are defined in the dependent claims. Methods are described herein but the methods are not claimed.

Described herein is a system including a humeral stem configured to be implanted within a humerus; a glenosphere configured to be mounted on a scapula; a humeral tray mounted to a proximal end of the humeral stem; an insert positioned on an outer surface of the humeral tray configured to articulate with the glenosphere; wherein the insert is not attached to the humeral tray such that the insert can articulate and move relative to the humeral tray.

The insert may include a convex articulation surface for articulating with the humeral tray and a concave articulation surface for articulating with the glenosphere, wherein the convex articulation surface has a greater radius than the concave articulation surface.

Optionally the insert and humeral tray are configured such that a CCD angle induced along a joint changes during joint motion.

Further described herein is a method which can include mounting a humeral tray to a proximal end of a humeral stem; mounting a glenosphere to a scapula; and positioning an insert between the humeral tray and the glenosphere such that the insert articulates with both the humeral tray and the glenosphere, wherein the insert is not attached to the humeral tray such that the insert can articulate and move relative to the humeral tray.

Furthermore, the humeral tray includes a constraint ridge located around a periphery of the humeral tray.

Optionally the insert has a height higher than a height of the constraint ridge.

These examples can be combined in any permutation or combination. This overview is intended to provide an overview of subject matter of the present patent application. It is not intended to provide an exclusive or exhaustive explanation of the invention, which is solely defined by the appended claims. The detailed description is included to provide further information about the present patent application.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 shows an implant system for a reverse shoulder arthroplasty, in accordance with one embodiment.
FIG. 2 shows top and side views of an insert for the reverse shoulder implant system, in accordance with one embodiment.
FIG. 3 shows top and side views of a humeral tray for the reverse shoulder implant system, in accordance with one embodiment.
FIG. 4 shows the reverse shoulder implant system with an arm at rest.
FIG. 5 shows the reverse shoulder implant system with an arm lifted.
FIG. 6 shows a side view of an insert, in accordance with one embodiment.
FIG. 7 shows a side view of an insert, in accordance with one embodiment.
FIG. 8 shows a side view of a humeral tray, in accordance with one embodiment.
FIG. 9 shows a method of improving a shoulder implant, the method not forming part of the present invention.

### DETAILED DESCRIPTION

As used herein, the following directional definitions apply. Anterior and posterior mean nearer the front or nearer the rear of the body, respectively, proximal and distal mean nearer to or further from the root of a structure, respectively, and medial and lateral mean nearer the sagittal plane or further from the sagittal plane, respectively. The sagittal plane is an imaginary vertical plane through the middle of the body that divides the body into right and left halves.

As noted above, reverse-type shoulder implant systems have been developed in which the conventional ball-and-socket configuration that replicates the natural anatomy of the shoulder is reversed, such that a concave recessed articulating component is provided at the proximal end of a humeral stem that articulates against a convex portion of a glenosphere of a glenoid component.

However, while the reverse-type shoulder design enables effective stabilization of the glenohumeral joint, it is often reported that the functional outcome is impaired and certain activities of daily living are no longer feasible. Therefore, current designs are often unable to give patients a full return to daily activities or offer the ability to perform subtle motions of the shoulder which are needed for a normal behavior of the glenohumeral joint.

The system described herein outlines a concept for an implant system, which is intended to enable a greater range of motion than offered with existing designs.

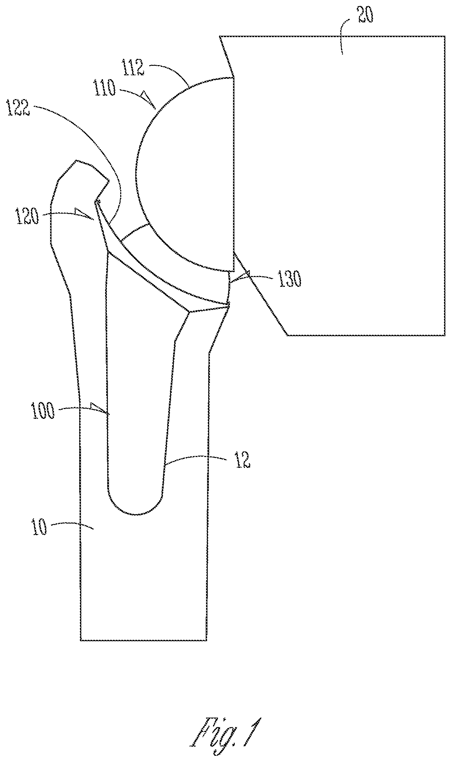

FIG. 1 shows an implant system for a reverse shoulder arthroplasty, in accordance with one embodiment. The system generally includes a humeral stem 100 configured to be implanted within a humerus 10, a glenosphere 110 configured to be mounted on a scapula 20, and having a curved outer surface 112, a humeral tray 120 mounted to a proximal end of the humeral stem 100, and an insert 130 positioned on an outer surface 122 of the humeral tray 120 and configured to articulate with the glenosphere 110. In this example, the insert 130 is not attached to the humeral tray 120 such that the insert 130 can articulate and move relative to the humeral tray 120. This can also be described that the insert 130 is "non-contained" relative to the humeral tray 120.

After implantation, the humeral tray 120 with the insert 130 can articulate about the glenosphere 112, such as to replicate the movement of the natural shoulder joint. The insert 130 is maintained in contact with the humeral tray 120 and the glenosphere 110 because of compressive loads across the joint, as well as conformity of the interface surfaces.

By having the insert be non-contained or not fixedly attached to the humeral tray 120 the insert 130 can travel along the humeral tray 120 during articulation. As will be further detailed below, this system allows the technical range of motion between the glenosphere 110 and the humeral stem 100 to be increased compared to a contained tray/insert concept where the humeral tray and insert do not move or articulate relative to each other. The extent of additional motion/freedom afforded with the present non-contained insert 130, as well as the resultant CCD angle, is based on the relative sizing of the insert and of the available space in the humeral tray.

Here, the humeral stem 100 can be sized or shaped or otherwise adapted to be fitted within a prepared proximal end canal of the humerus 10. In an example, the humeral stem 100 can be a metallic device anchored into the humeral canal of the humerus 10 using cement or a press-fit, for example.

The glenosphere 110 can be sized or shaped or otherwise configured to be mounted to a prepared surface of a patient's glenoid, such as via a plurality of screws.

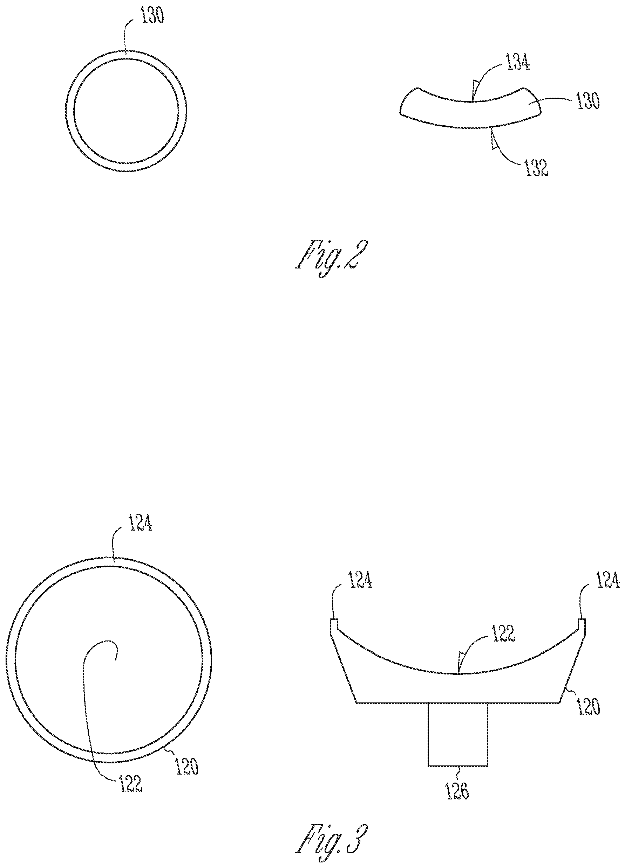

FIG. 2 shows top and side views of the insert 130, in accordance with one embodiment; and FIG. 3 shows top and side views of the humeral tray 120.

In general, the insert 130 can be formed of a polymeric material, such as polyethylene and has a circular shape which is of a smaller diameter than the diameter of a concave articulation surface 122 of the humeral tray 124. The humeral tray 120 can be formed of a metallic material such as CoCr or a titanium alloy, for example, the humeral tray 120 includes the concave articulation surface 122 which receives the insert 130.

The humeral tray 120 includes a constraint ridge 124 located around a periphery of the humeral tray 120. The height of the constraint ridge 124 and the height of the insert 130 are chosen such that the insert 130 is constrained from moving past the constraint ridge 124. Moreover, the insert 130 can have a height higher than a height of the constraint ridge 124. This minimizes notching during articulation of the joint.

The insert 130 can include a convex articulation surface 132 for articulating with the humeral tray 120 and a concave articulation surface 134 for articulating with the glenosphere 110, wherein the convex articulation surface 132 has a greater radius than the concave articulation surface 134.

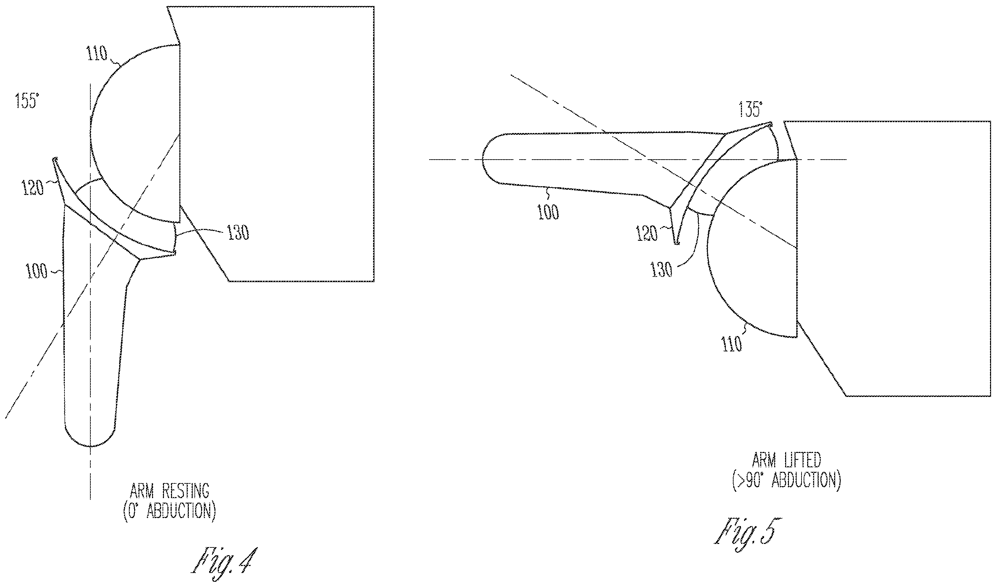

FIG. 4 shows the reverse shoulder implant system with an arm at rest; and FIG. 5 shows the reverse shoulder implant system with an arm lifted.

FIGs 4 and 5 help shows the features of the present system. Here, due to the ability of the non-contained insert 130 to articulate and move with respect to its interface with the humeral tray 120, the CCD angle induced across the joint changes during motion.

For example, while the arm is resting at the side of the body (0° abduction) the resultant CCD angle would be relatively flatter (>150°) (FIG. 4), offering a high level of supero-inferior constraint. During abduction, the insert 130 can rotate with respect to both the glenosphere 110 and with respect to the humeral tray 120, reducing the CCD angle (≤135°) and offering a greater potential range of motion (FIG. 5).

Due to the ability of the non-contained insert 130 to articulate and move with respect to its interface with the humeral tray 120, the technical range of motion between the glenosphere 110 and the humeral stem 100 is increased compared to a contained tray/insert system. The extent of additional motion/freedom afforded with the non-contained insert 130, as well as the resultant CCD angle, is based on the relative sizing of the insert and of the available space in the humeral tray. Thus, different results can be provided by providing a smaller or larger insert 130 or humeral tray 120.

In the present system, beyond the additional technical range of motion that is afforded, there is expected to be additional scope for the patient to better perform activities of daily living due to the freedom to subtly alter the relative positions of the insert 130 and the humerus tray 120 during minor motions. These small motions, while constrained by the conformity of the articulations and bounded by the humeral tray 120, give the patient greater control over their ability to move the arm as they need it and account for the patient specific nature of glenohumeral rhythm.

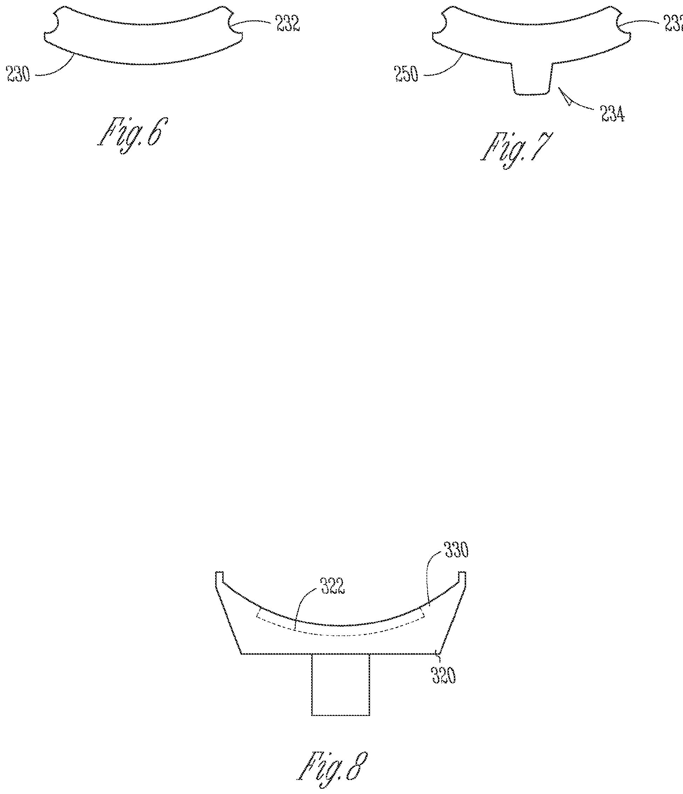

FIG. 6 shows a side view of an insert 230, in accordance with one embodiment. In this example, the insert 230 can optionally include a circumferential groove 232 around an outer periphery of the insert 230. The groove 232 helps resist lift-off of the insert 230 from the humeral tray 120 at the extremes of insert 230 movement across the tray 120.

FIG. 7 shows a side view of an insert 250, in accordance with one embodiment; and FIG. 8 shows a side view of a humeral tray 320, in accordance with one embodiment. In this example, the humeral tray 320 can include a cut-out 322 in a central surface 330 of the humeral tray 320. The insert 250 can include a post 234 extending from a bottom surface of the insert 250. The post 234 is configured to ride in the cut-out 322 so as to prevent excessive motion of the insert 250 relative to the humeral tray 320.

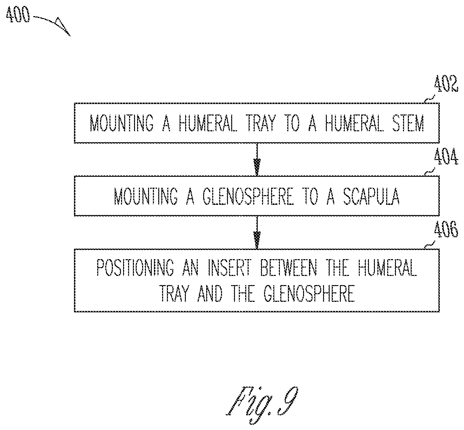

FIG. 9 shows a method 400 of improving a shoulder implant, the method not forming part of the present invention.

Method 400 includes mounting a humeral tray to a proximal end of a humeral stem (402). This can be done after the humeral stem is properly implanted in the humeral canal of the humerus. Method 400 further includes mounting a glenosphere to a scapula (404). As noted above, this can be accomplished in a variety of fashions, as by using a base plate, or a cement fit, or with screws or other fasteners. The method further includes positioning an insert (406) between the humeral tray and the glenosphere such that the insert articulates with both the humeral tray and the glenosphere. In the example method 400, the insert is not attached to the humeral tray such that the insert can articulate and move relative to the humeral tray.

In some options the method can include any of the other features discussed above such as the humeral tray including a constraint ridge located around a periphery of the humeral tray, and the insert having a height higher than a height of the constraint ridge.

The present system allows for better joint movement after a reverse-type shoulder implant. By providing the non-contained insert, the system allows patients find that the functional outcome is improved relative to a contained insert implant.

### Additional Notes

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein."

Moreover, in the following claims, the terms "first," "second," and "third," etc. are used merely as labels, and are not intended to impose numerical requirements on their objects.

## Claims

1. An apparatus comprising:
a humeral tray (120, 320) configured to be mounted to a proximal end of a humeral stem (100); and
an insert (130, 230,250) positioned on an outer surface (122) of the humeral tray (120, 320) configured to articulate with a glenosphere (110);
wherein the insert (130, 230, 250) is not attached to the humeral tray (120, 320) such that the insert (130, 230, 250) can articulate and move relative to the humeral tray (120, 320),
**characterized in that** the humeral tray (120, 320) includes a constraint ridge (124) located around a periphery of the humeral tray (120, 320), wherein the height of the constraint ridge (124) and the height of the insert are such that the insert (130) is constrained from moving past the constraint ridge (124); and
**in that** the insert (130, 230, 250) includes a circular shape which is of a smaller diameter than the outer surface (122) of the humeral tray (120, 320).

2. The apparatus of claim 1, wherein the humeral tray (120, 320) includes a concave articulation surface (122).

3. The apparatus of claim 1, wherein the insert (230) includes a circumferential groove (232) around an outer periphery of the insert (230).

4. The apparatus of claim 1, wherein the insert (130, 230, 250) has a height higher than a height of the constraint ridge (124).

5. The apparatus of claim 1, wherein the humeral tray (320) includes a cut-out (322) in a central surface (330) of the humeral tray (320) and wherein the insert (250) includes a post (234) extending from a bottom surface of the insert (250), wherein the post (234) is configured to ride in the cut-out (322) so as to prevent excessive motion of the insert (250) relative to the humeral tray (320).

6. The apparatus of claim 1, wherein the insert (130) includes a convex articulation surface (132) for articulating with the humeral tray (120) and a concave articulation surface (134) for articulating with the glenosphere (110), wherein the convex articulation surface (132) has a greater radius than the concave articulation surface (134).

7. A system comprising:
the apparatus of claim 1; and
a glenosphere (110) configured to be mounted on a scapula (20).

8. The system of claim 7, wherein the humeral tray (120, 320) includes a concave articulation surface (122).

9. The system of claim 7, wherein the insert (230) includes a circumferential groove (232) around an outer periphery of the insert (230).

10. The system of claim 9, wherein the insert (230) has a height higher than a height of the constraint ridge (124).

11. The system of claim 7, wherein the humeral tray (320) includes a cut-out (322) in a central surface (330) of the humeral tray (320) and wherein the insert (250) includes a post (234) extending from a bottom surface of the insert (250), wherein the post (234) is configured to ride in the cut-(322) out so as to prevent excessive motion of the insert relative to the humeral tray.

## Patentansprüche

1. Vorrichtung, umfassend:
eine Humerusschale (120, 320), die konfiguriert ist, um an einem proximalen Ende eines Humerusschafts (100) montiert zu werden; und
einen Einsatz (130, 230,250), der an einer Außenfläche (122) der Humerusschale (120, 320) positioniert und konfiguriert ist, um mit einer Glenosphäre (110) zu artikulieren;
wobei der Einsatz (130, 230, 250) nicht an der Humerusschale (120, 320) angebracht ist, sodass der Einsatz (130, 230, 250) gelenkig und in Bezug auf die Humerusschale (120, 320) beweglich ist,
**dadurch gekennzeichnet, dass**
die Humerusschale (120, 320) einen Beschränkungsgrat (124) beinhaltet, der sich um einen Umfang der Humerusschale (120, 320) befindet, wobei die Höhe des Beschränkungsgrats (124) und die Höhe des Einsatzes derart sind, dass der Einsatz (130) daran gehindert wird, sich über den Beschränkungsgrat (124) hinaus zu bewegen; und
dass der Einsatz (130, 230, 250) eine kreisförmige Form beinhaltet, die von einem kleineren Durchmesser als die Außenfläche (122) der Humerusschale (120, 320) ist.

2. Vorrichtung nach Anspruch 1, wobei die Humerusschale (120, 320) eine konkave Gelenkfläche (122) beinhaltet.

3. Vorrichtung nach Anspruch 1, wobei der Einsatz (230) eine Umfangsrille (232) um einen Außenumfang des Einsatzes (230) beinhaltet.

4. Vorrichtung nach Anspruch 1, wobei der Einsatz (130, 230, 250) eine Höhe aufweist, die höher ist als die Höhe des Beschränkungsgrats (124).

5. Vorrichtung nach Anspruch 1, wobei die Humerusschale (320) einen Ausschnitt (322) in einer mittleren Fläche (330) der Humerusschale (320) beinhaltet, und wobei der Einsatz (250) einen Pfosten (234) beinhaltet, der sich von einer Bodenfläche des Einsatzes (250) erstreckt, wobei der Pfosten (234) konfiguriert ist, um in dem Ausschnitt (322) zu laufen, um eine übermäßige Bewegung des Einsatzes (250) in Bezug auf die Humerusschale (320) zu verhindern.

6. Vorrichtung nach Anspruch 1, wobei der Einsatz (130) eine konvexe Gelenkfläche (132) zum Artikulieren mit der Humerusschale (120) und eine konkave Gelenkfläche (134) zum Artikulieren mit der Glenosphäre (110) beinhaltet, wobei die konvexe Gelenkfläche (132) einen größeren Radius aufweist als die konkave Gelenkfläche (134).

7. System, umfassend:
die Vorrichtung nach Anspruch 1; und
eine Glenosphäre (110), die konfiguriert ist, um an einem Schulterblatt (20) montiert zu werden.

8. System nach Anspruch 7, wobei die Humerusschale (120, 320) eine konkave Gelenkfläche (122) beinhaltet.

9. System nach Anspruch 7, wobei der Einsatz (230) eine Umfangsrille (232) um einen Außenumfang des Einsatzes (230) beinhaltet.

10. System nach Anspruch 9, wobei der Einsatz (230) eine Höhe aufweist, die höher ist als die Höhe des Beschränkungsgrats (124).

11. System nach Anspruch 7, wobei die Humerusschale (320) einen Ausschnitt (322) in einer mittleren Fläche (330) der Humerusschale (320) beinhaltet, und wobei der Einsatz (250) einen Pfosten (234) beinhaltet, der sich von einer Bodenfläche des Einsatzes (250) erstreckt, wobei der Pfosten (234) konfiguriert ist, um in dem Ausschnitt (322) zu laufen, um eine übermäßige Bewegung des Einsatzes in Bezug auf die Humerusschale zu verhindern.

## Revendications

1. Appareil comprenant :
un plateau huméral (120, 320) conçu pour être monté sur une extrémité proximale d'une tige humérale (100) ; et
un insert (130, 230, 250) positionné sur une surface externe (122) du plateau huméral (120, 320) conçu pour s'articuler avec une glénosphère (110) ;
ledit insert (130, 230, 250) n'étant pas fixé au plateau huméral (120, 320) de sorte que l'insert (130, 230, 250) puisse s'articuler et se déplacer par rapport au plateau huméral (120, 320),
**caractérisé en ce que** le plateau huméral (120, 320) comprend une crête de contrainte (124) située autour de la périphérie du plateau huméral (120, 320), ladite hauteur de la crête de contrainte (124) et ladite hauteur de l'insert étant telles que l'insert (130) est contraint de se déplacer au-delà de la crête de contrainte (124) ; et
**en ce que** l'insert (130, 230, 250) comprend une forme circulaire qui est d'un diamètre plus petit que la surface externe (122) du plateau huméral (120, 320).

2. Appareil selon la revendication 1, ledit plateau huméral (120, 320) comprenant une surface d'articulation concave (122).

3. Appareil selon la revendication 1, ledit insert (230) comprenant une rainure circonférentielle (232) autour de la périphérie externe de l'insert (230).

4. Appareil selon la revendication 1, ledit insert (130, 230, 250) comportant une hauteur supérieure à la hauteur de la crête de contrainte (124).

5. Appareil selon la revendication 1, ledit plateau huméral (320) comprenant une découpe (322) dans une surface centrale (330) du plateau huméral (320) et ledit insert (250) comprenant un montant (234) s'étendant depuis une surface inférieure de l'insert (250), ledit montant (234) étant conçu pour se déplacer dans la découpe (322) de manière à empêcher un mouvement excessif de l'insert (250) par rapport au plateau huméral (320).

6. Appareil selon la revendication 1, ledit insert (130) comprenant une surface d'articulation convexe (132) destinée à s'articuler avec le plateau huméral (120) et une surface d'articulation concave (134) destinée à s'articuler avec la glénosphère (110), ladite surface d'articulation convexe (132) comportant un rayon supérieur à celui de la surface d'articulation concave (134).

7. Système comprenant :
l'appareil selon la revendication 1 ; et
une glénosphère (110) conçue pour être montée sur une omoplate (20).

8. Système selon la revendication 7, ledit plateau huméral (120, 320) comprenant une surface d'articulation concave (122).

9. Système selon la revendication 7, ledit insert (230) comprenant une rainure circonférentielle (232) autour de la périphérie externe de l'insert (230).

10. Système selon la revendication 9, ledit insert (230) comportant une hauteur supérieure à la hauteur de la crête de contrainte (124).

11. Système selon la revendication 7, ledit plateau huméral (320) comprenant une découpe (322) dans une surface centrale (330) du plateau huméral (320) et ledit insert (250) comprenant un montant (234) s'étendant depuis une surface inférieure de l'insert (250), ledit montant (234) étant conçu pour se déplacer dans la découpe (322) de manière à empêcher un mouvement excessif de l'insert par rapport au plateau huméral.
